# EUROPEAN PATENT SPECIFICATION

(11) **EP 0 661 992 B1**
(45) Date of publication and mention of the grant of the patent: **07.01.2004**
(21) Application number: 93921651.1
(22) Date of filing: 17.09.1993
(51) Int. Cl.: A61K 38/16, A61K 47/26, A61K 47/10

(54) **PHARMACEUTICAL FORMULATIONS OF INTERLEUKIN-1 INHIBITORS**
PHARMAZEUTISCHE ZUSAMMENSETZUNGEN MIT INTERLEUKIN-1 INHIBITOREN
COMPOSITIONS PHARMACEUTIQUES A BASE D'INHIBITEURS DE L'INTERLEUKINE-1

(30) Priority: 17.09.1992 US 947006
(43) Date of publication of application: 12.07.1995
(73) Proprietor: Amgen Inc., Thousand Oaks, CA 91320-1799 (US)
(72) Inventor: SABADOS, Benjamin K., Broomfield, CO 80020 (US)
(74) Representative: Grünecker, Kinkeldey, Stockmair & Schwanhäusser Anwaltssozietät
(86) International application number: PCT/US1993/008802
(87) International publication number: WO 1994/006457

(56) References cited:
- WO-A-92/12724
- DATABASE WPI Week 7547, Derwent Publications Ltd., London, GB; AN 75-77526 & JP,A,49 054 514 (TOYO BREWING KK)
- DATABASE WPI Week 7547, Derwent Publications Ltd., London, GB; AN 75-77525 & JP,A,49 054 513 (TOYO BREWING KK)

## Description

### Background of the Invention

The present invention relates to pharmaceutical compositions, and more specifically to pharmaceutical formulations of interleukin-1 inhibitors.

Interleukin-1 inhibitors, as described in U.S. Patent No. 5,075,222, are useful in the treatment of interleukin-1 mediated diseases. Such interleukin-1 inhibitors are also known as interleukin-1 receptor antagonists (IL-1ra). Interleukin-1 mediated diseases include rheumatoid arthritis (RA), inflammatory bowel disease (IBD), sepsis, sepsis syndrome, osteoporosis, ischemic injury, graft vs. host disease, reperfusion injury, asthma, insulin diabetes, myelogenous and other leukemias, psoriasis and cachexia. These and other inflammatory diseases are characterized by the production of cytokines, including interleukin-1.

Cytokines are extracellular proteins that modify the behavior of cells, particularly those cells in the immediate area of cytokine synthesis and release. Many of these cytokines are manufactured by cells of the macrophage/monocyte lineage. For example, rheumatoid arthritis is an autoimmune disease characterized by a chronic inflammatory process primarily involving the synovial membrane of peripheral joints as reported in Harris, N. Engl. J. Med. 322:1277 (1990). The large majority of mononuclear cells present in the joint fluid of RA patients are activated monocytes/macrophages and T lymphocytes.

Classic therapies for the treatment of RA and IBD include indomethacin and other non-steroidal anti-inflammatory drugs (NSAIDs) and salicylates. Interleukin-1 receptor antagonist has also been demonstrated to be a useful therapeutic in the treatment of rheumatoid arthritis. Sepsis and septic shock have been treated with therapies such as vasoactive drugs, antibiotics, β-receptor stimulants including isopretenol and dopamine, and α-receptor blocking agents, for example, phenoxybenzamine and phentolamine. Osteoporosis has been treated with estrogen, vitamin D, and fluoride. Ischemic injury has classically been treated with anticoagulants and antiplatelet compounds. Asthma has been treated with a wide variety of therapies including a or β adrenergic stimulants to dilate airways, methylxanthines to improve movement of airway mucus, glucocorticoids to reduce airway inflammation, cromolyn sodium to inhibit degranulation of mast cells, and anticholinergics for bronchodilation.

All of the treatments for these diseases have various problems. For example, even using the current known therapeutics, the death rate from septic shock is about 40-50%. Thus, there is a need for new therapeutics such as interleukin-1 receptor antagonist for treating such conditions and for formulations for delivering such therapeutics in acceptable ways.

Australian Patent Application AU 9173636 and Canadian Patent application 2039458 disclose the use of interleukin-1 receptor antagonist in the above-listed interleukin-1 mediated diseases. The IL-1ra formulation used in these references is a solution containing 10 mM sodium phosphate (pH 7.0), 150 mM NaCl, 0.1 mM EDTA (ethylene diamine tetraacetic acid). In this formulation the material remains stable for only about two weeks under normal refrigeration (4°-8°C). Because of the necessity to ship and store IL-1ra for use in treating IL-1 mediated conditions for an extended period, a need exists for a more stable formulation.

Polysorbate 80, also known as polyoxyethylene sorbitan monooleate or Tween 80, is a nonionic biological detergent or surfactant which can be used for a variety of purposes, including emulsifying, stabilizing and dispersing. Non-ionic surfactants such as polysorbate-80 are also added to certain protein formulations to reduce aggregation and denaturation, as well as for increased solubility. Polysorbate 80 has been used to stabilize a variety of compounds. U.S. Patent No. 4,156,777 describes a process for producing glucopyranose-nitrosurea compounds utilizing polysorbate 80 as a stabilizer. U.S. Patent No. 4,816,459 also describes the use of polysorbate 80 as a stabilizer for tetrazolyl-substituted pyrido [1,2-a] pyrimidines. U.S. Patent No. 5,032,574 utilizes polysorbate 80 to solubilize or disperse the active ingredient in the pharmaceutical composition of an antimicrobial peptide of 3700 Daltons, while U.S. Patent No. 5,073,378 describes the use of polysorbate 80 as an emulsifying agent for collagen products. However, it is difficult to predict the effect a particular stabilizing agent will have on the stability of a particular protein. For example, a stabilizing agent's interaction with a protein may cause a protein to degrade rather than the desired effect of reducing degradation.

Accordingly, a need exists to identify formulations that stabilize IL-1 inhibitors. The present invention satisfies this need and provides related advantages as well.

### Summary of the Invention

Compositions comprising interleukin-1 inhibitors, buffers and nonionic surfactants or viscosity enhancers are provided which are suitable for use as stable pharmaceutical formulations. These compositions are suitable for intra-articular, intravenous, intramuscular, subcutaneous, intra-dermal, intrathecal, intraventricular (CNS), topical or oral administration or for use as suppositories, enemas, or inhaled aerosols.

### Detailed Description of the Invention

The present invention relates to pharmaceutical compositions containing an IL-1 inhibitor and a non-ionic surfactant or a viscosity enhancer. IL-1ra has demonstrated susceptibility to agitation. Agitated vials of purified bulk concentrate IL-1ra form precipitates that subsequently fail to meet desirable standards for appearance and particulates. In order to prevent precipitation that leads to undesirable aggregation, a modification to the formulation was sought.

In one embodiment, the pharmaceutical compositions contain an IL-1 inhibitor, particularly IL-1ra, and a non-ionic surfactant. A non-ionic surfactant is a surface active agent whose solubilizing contribution can be supplied by a chain of ethylene oxide groups. A surfactant changes the properties of a solvent in which it is dissolved to a much greater extent than would be expected from its concentration. Hydrophilicity in nonionic surfactants is provided by hydrogen bonding with water molecules. Oxygen atoms and hydroxyl groups readily form strong hydrogen bonds, whereas ester and amide groups form hydrogen bonds less readily. Hydrogen bonding provides solubilization in neutral and alkaline media. In a strongly acid environment, oxygen atoms are protonated, providing a quasi-cationic character. Each oxygen atom makes a contribution to water solubility. More than a single oxygen atom is therefore needed to solubilize a nonionic surfactant in water. Nonionic surfactants are compatible with ionic and amphoteric surfactants. Since a polyoxytheylene group can easily be introduced by reaction of ethylene oxide with any organic molecule containing an active hydrogen atom, a wide variety of structures can be solubilized by the oxylation. (Encyclopedia of Chemical Technology, 3rd Ed. Vol. 22 p. 360).

The surfactant also serves to reduce aggregation and denaturation of proteins. "Aggregation", as used herein, means the formation of a complex of many protein molecules (i.e., as in the clumping together of several proteins). "Denaturation", as used herein, means the loss of the secondary and tertiary structure of a protein, which usually correlates with a loss of bioactivity. By reducing aggregation, physical degradation due to changes in surface charges is also reduced. It is believed that the non-ionic surfactant serves to block the air-liquid interface thus preventing denaturation of the protein at this interface. In addition, the use of non-ionic surfactants permits the composition to be exposed to shear surface stresses without causing denaturation of the protein. Further, such surfactant containing formulations may be employed in aerosol devices such as those used in pulmonary dosing, and needleless jet injector guns.

Suitable non-ionic surfactants useful in the pharmaceutical compositions of the present invention include, for example, block copolymers of ethylene oxide and propylene oxide, block copolymers of propylene oxide and ethylene oxide, sorbitan monolaurate, sorbitol ester, polyglycerol fatty acid ester, cocamide DEA lauryl sulfate, alkanolamide, polyoxyethylene propylene glycol stearate, polyoxyethylene lauryl ether, polyoxyethylene cetyl ether, polysorbate, glycerol monostearate, glycerol distearate, sorbitol monopalmitate, polyoxyethylene sorbitan monooleate, polyoxyethylene sorbitan monolaurate and propylene glycol monostearate. For example, agitation studies comparing control IL-1ra to that containing 0.1% Polysorbate (Tween) 80 demonstrated that IL-1ra in the buffer containing 0.1% Polysorbate 80 had background values similar to non-agitated controls, while the IL-1ra in the formulation without Polysorbate 80 exhibited profound precipitation. The degree of precipitation was measured by addition of 150 µl of the test article per well in a 96 well plate, and measuring the turbidity at 405 nm. These agitation studies provided the rationale for using the formulation containing Polysorbate 80. Other surfactants were also tested for their ability to stabilize the formulation against agitation as described in the Examples below.

All compositions tested were shown to exhibit resistance to physical degradation and precipitation upon agitation. Also, it was found that with higher concentrations of interleukin-1 inhibitor at concentrations below 0.1%, proportionately more non-ionic surfactant was used to stabilize the composition. Concentrations in the order of 0.1% by weight of polysorbate-80 are preferred to stabilize the composition.

In a further embodiment of the present invention, the pharmaceutical compositions of the present invention contain an IL-1ra inhibitor, particularly IL-1ra, and a viscosity enhancer. A viscosity enhancer is a substance that acts as a thickening agent to increase the viscosity of a composition. A viscosity enhancer is believed to prevent IL-1ra molecules from interacting with each other or the air=liquid interface that can lead to physical degradation.

Viscosity enhancers suitable in the present invention include, for example, polyethylene glycol (PEG) , hydroxyl propyl cellulose, and carrageenan gum. In experimental studies, PEG was evaluated for its ability to prevent precipitation and aggregation. Concentrations of up to about 2% PEG were found to be effective in preventing precipitation.

The pharmaceutical compositions of the present invention also contain a buffer to maintain the pH at a desired biological level. Any non-toxic buffers can be used for this purpose. Useful buffers include, for example, phosphate buffers, citrate buffers and acetate buffers.

Once the therapeutic composition has been formulated, it may be stored in sterile vials as a solution, suspension, gel, emulsion, solid, or dehydrated or lyophilized powder. Such formulations may be stored either in a ready to use form or requiring reconstitution immediately prior to administration. The preferred storage of such formulations is at temperatures approximating refrigerated storage conditions or frozen. It is also preferred that such formulations containing Il-1ra are stored and administered at or near physiological pH. It is presently believed that storage and administration in a formulation at a high pH (i.e. greater than 8) or at a low pH (i.e. less than 5) is undesirable.

Preferably, the manner of administering the formulations containing IL-1ra is via an intra-articular, subcutaneous, intra-dermal, intrathecal, intraventricular (CNS), intramuscular, intravenous, topical or oral route, or as a suppository, enema, or inhaled aerosol. To achieve and maintain the desired level of IL-1ra in the body, repeated doses may be administered. All of these methods are intended to create a specified concentration range of IL-1ra in the patient's blood stream, or in other body tissues or fluids. For example, it is believed that the maintenance of circulating blood plasma concentrations of IL-1ra of less than 0.01 ng per ml of plasma may indicate an ineffective composition while the prolonged maintenance of circulating levels in excess of 100 µg per ml may indicate a composition with undesirable side effects.

As indicated above, it is also contemplated that certain formulations containing IL-1ra are to be administered orally. Preferably, IL-1ra which is administered in this fashion is enteric or polymeric coated. The enteric or polymeric coated IL-1ra may be formulated with or without those carriers customarily used in the compounding of solid dosage forms. Preferably, the material is designed so that the active portion of the formulation is released at that point in the gastrointestinal tract when bioavailability is maximized and pre-systemic degradation is minimized. The bioavailability is expected to be decreased by pre-systemic degradation, hence oral doses will be greater than those listed above. Additional excipients may be included to facilitate absorption of the IL-1ra. Diluents, flavorings, low melting point waxes, vegetable oils, lubricants, suspending agents, tablet disintegrating agents, and binders may also be employed.

A preferred subcutaneous dosage for the treatment of interleukin-1 mediated arthritis should produce blood IL-1ra concentrations between 1 and 1000 ng/ml. Accordingly, it is preferred that, initially, doses are administered to bring the circulating levels of IL-1ra above 10 ng per ml of plasma and that, thereafter, doses are administered at a suitable frequency to keep the circulating level of IL-1ra at or above approximately 10 ng per ml of plasma. The frequency of dosing will depend on pharmacokinetic parameters describing the absorption of subcutaneous IL-1ra from the formulation. The frequency of dosing can be 1-10 times per day, or less frequent than daily in the case of a sustained or timed release dosage form.

A preferred dosage range for the treatment of interleukin-1 mediated IBD is between about 0.5-50 mg per kg of patient weight administered between about 1 and 10 times per day, or less. In a more preferred embodiment the dosage is between about 1-10 mg per kg of patient weight administered between about 3 and 5 times per day. The frequency of dosing will depend on pharmacokinetic parameters describing absorption of IL-1ra from the formulation used. When used for the treatment of interleukin-1 mediated IBD, the administration of IL-1ra can also be accomplished in a suitably formulated enema.

A preferred dosage range for the treatment of interleukin-1 mediated septic shock is between about 10 to 120 mg per kg per day of patient body weight per 24 hours administered by continuous intravenous infusion. In a more preferred embodiment the dosage is between about 1-2 mg per kg per hour of patient body weight administered intravenously by continuous infusion.

A preferred dosage range for the treatment of interleukin-1 mediated ischemia and reperfusion injury is between about 1-50 mg per kg of patient weight administered hourly. In a preferred embodiment an initial bolus of about 15-50 mg per kg of Il-1ra is administered, followed by hourly injections of about 5-20 mg per kg. The frequency of dosing will depend on pharmacokinetic parameters of the IL-1ra for the formulation used.

Regardless of the manner of administration, the specific dose is calculated according to the approximate body weight or surface area of the patient. Further refinement of the calculations necessary to determine the appropriate dosage for treatment involving each of the above mentioned formulations is routinely made by those of ordinary skill in the art and is within the ambit of tasks routinely performed by them without undue experimentation. These dosages may be ascertained through use of the established assays for determining dosages utilized in conjunction with appropriate dose-response data and pharmacokinetic data.

It should be noted that the IL-1ra formulations described herein may be used for veterinary as well as human applications and that the term "patient" should not be construed in a limiting manner. In the case of veterinary applications, the dosage ranges should be the same as specified above.

The following examples demonstrate the effectiveness of these formulations which have been prepared and tested and have been found to satisfy the objectives set forth in accordance with the present invention.

The control for the following experiments was prepared by measuring the optical density at 405 nm of different concentrations of recombinant human IL-1ra (rhIL-1ra) after varying periods of agitation. The results are set forth in Table 1.

**TABLE 1**

| **CONTROL** | | | | |
|---|---|---|---|---|
| **Time (Hours)** | **IL-1ra Concentration (mg/ml)** | | | |
| | **200** | **150** | **100** | **50** |
| 0 | 0.062 | 0.060 | 0.070 | 0.051 |
| 1.75 | 0.0171 | 0.099 | 0.089 | 0.066 |
| 8 | 0.692 | 0.694 | 0.498 | 0.371 |
| 12 | 0.967 | 0.765 | 0.560 | 0.258 |

### EXAMPLE 1

### Preparation of Formulation A

Preparation of 10 mM EDTA. 0.93 g EDTA was placed into a 250 ml tared bottle. 200 g of sterile water for injection was added thereto. The contents of the bottle were stirred and the pH was adjusted to 6.5 ± .02 using NaOH and qs to 250 g.

Preparation of Buffer. 900 g of sterile water for injection was added to a 1 liter tared bottle. To this was added 8.20 g NaCl (Sigma, St. Louis, MO), 2.86 g of sodium citrate dihydrate (Sigma, St. Louis, MO), and 0.058 g citric acid monohydrate (Sigma, St. Louis, MO). To this mixture was added 50 g of the 10 mM EDTA as prepared above. This final mixture was stirred until all solids were dissolved. The pH was adjusted to 6.5 ± .02 using NaOH., and q.s. to 1000g.

Preparation of Formulation. Purified bulk concentrate of rhIL-1ra was removed from -70°C and allowed to thaw at room temperature. Purified bulk concentrate of rhIL-lra prepared according to the process disclosed in PCT published application WO 91/08285 of Hageman et al., which is incorporated herein by reference. The material so produced is concentrated at approximately 190 - 250 mg/ml in 10 mM sodium citrate, 140 mM sodium chloride, 0.5 mM EDTA at pH 6.5.

Buffer as prepared above was added to adjust the concentration. Several concentrations were examined, including: 200 mg/ml, 100 mg/ml, 80 mg/ml, 70 mg/ml, 50 mg/ml, and 20 mg/ml. Tween 80 (Spectrum, Lot D1014) was added to yield a total weight concentration of Tween 80 of 0.01%. Concentrations of Tween 80 from 0.01% to 1.0% fall within the scope of this invention.

Stability of Formulation A. The stability of this formulation to agitation was tested by vortexing the various concentrations for varying amounts of time. After vortexing the formulation for the designated period of time, the optical density of the solution was measured at 405 nm. using a kinetic microplate reader (Molecular Devices). A formulation is considered stable when optical density measurements at 405 nm are less than approximately 0.15. When optical density measurements at 405 nm are 0.15 or greater, turbidity begins to become observable due to the presence of solid particles, and this formulation is considered unstable . The results of this experiment are set forth in Table 2. This experiment demonstrates that 0.1% Tween 80 provides stability against agitation for IL-1ra formulations ranging from 20 - 200 mg/ml.

**TABLE 2**

| **0.1% (by weight) Tween 80 IL-1ra 20-200 mg/ml** | | | | | | |
|---|---|---|---|---|---|---|
| **Time (Hours)** | **IL-ira Concentration (mg/ml)** | | | | | |
| | **200** | **100** | **80** | **70** | **50** | **20** |
| 0 | 0.057 | 0.048 | 0.051 | 0.046 | 0.046 | 0.043 |
| 8 | 0.057 | 0.054 | 0.051 | 0.048 | 0.046 | 0.044 |
| 16 | 0.057 | 0.051 | 0.052 | 0.048 | 0.046 | 0.043 |
| 24 | | 0.054 | 0.053 | 0.048 | 0.045 | 0.044 |

Clinical Results. A randomized, double-blind, placebo-controlled phase II trial of IL-1ra in Formulation A was conducted by 63 centers from eight countries. The trial included 901 patients who had sepsis syndrome with evidence of hypotension and/or end organ dysfunction. Patients were randomly allocated to one of three groups: placebo, IL-1ra (100 mg loading dose followed by intravenous infusion of 1.0 mg/kg/hr for 72 hours) or IL-1ra (100 mg loading dose followed by intravenous infusion of 2.0 mg/kg/hr for 72 hours).

Of the 901 patients randomized, 298 received IL-1ra (1.0 mg/kg/hr), 293 received IL-1ra (2.0 mg/kg/hr) and 302 received placebo. A retrospective analysis of the results showed that IL-1ra does provide a survival benefit as a function of increasing predicted risk for mortality in patients with sepsis syndrome.

The risk of mortality was quantified using a risk prediction model developed from databases independently of the phase III trial. The model was validated and found to be useful for predicting the placebo mortality rate in the phase III trial. Data for derivation of predicted risk of mortality were available for 892 of 893 patients. Results showed that IL-1ra produced a statistically significant survival benefit in patients with a predicted risk for mortality of ≥24% (p=0.032). In these patients, IL-1ra reduced mortality by 22% compared to placebo.

### EXAMPLE 2

### Preparation of Formulation B

EDTA and buffer were prepared as set forth in Example 1.

The formulation was prepared as set forth in Example 1, except that only one concentration (100 mg/ml) was used. The non-ionic surfactant used was Tween 20 (Spectrum).
The results of this experiment are set forth in Table 3. This experiment demonstrates that Tween 20 at concentrations from 0.01 weight percent to 1.0 weight percent provides stability against agitation for IL-1ra formulations.

**TABLE 3**

| **0.001 to 1.0% (by weight) Tween 20 IL-1ra 100 mg/ml** | | | | | |
|---|---|---|---|---|---|
| **Time (Hours)** | **Weight percent of Tween 20** | | | | |
| | **0.001** | **0.01** | **0.1** | **0.5** | **1.0** |
| 0 | .061 | .056 | .058 | .059 | .063 |
| 1.75 | .113 | .052 | .051 | .061 | .071 |
| 8 | .667 | .075 | .063 | -- | -- |
| 12 | .580 | .075 | .064 | -- | -- |

### EXAMPLE 3

### Preparation of Formulation C

EDTA and buffer were prepared as set forth in Example 1.

The formulation was prepared as set forth in Example 1, except that only one concentration (100 mg/ml) was used. The non-ionic surfactant used was Pluronic 108 (BASF). The results of this experiment are set forth in Table 4. This experiment demonstrates that Pluronic 108 at concentrations from 0.01 weight percent to 1.0 weight percent provides stability against agitation for IL-1ra formulations.

**TABLE 4**

| **0.001 to 1.0% (by weight) Pluronic 108 IL-1ra 100 mg/ml** | | | | | |
|---|---|---|---|---|---|
| **Time (Hours)** | **Weight percent of Pluronic 108** | | | | |
| | **0.001** | **0.01** | **0.1** | **0.5** | **1.0** |
| 0 | .059 | .057 | .057 | .061 | .065 |
| 1.75 | .056 | .084 | .052 | .065 | .066 |
| 8 | .134 | .071 | .069 | -- | -- |
| 12 | .048 | .069 | .068 | -- | -- |

### EXAMPLE 4

### Preparation of Formulation D

EDTA and buffer were prepared as set forth in Example 1.

The formulation was prepared as set forth in Example 1, except that only one concentration (100 mg/ml) was used. The non-ionic surfactant used was Pluronic F68 (BASF).
The results of this experiment are set forth in Table 5. This experiment demonstrates that Pluronic F68 at concentrations from 0.01 weight percent to 1.0 weight percent provides stability against agitation for IL-1ra formulations.

**TABLE 5**

| **0.001 to 1.0% (by weight) Pluronic F68 IL-1ra 100 ng/ml** | | | | | |
|---|---|---|---|---|---|
| **Time (Hours)** | **Weight percent of Pluronic F68** | | | | |
| | **0.001** | **0.01** | **0.1** | **0.5** | **1.0** |
| 0 | .066 | .064 | .070 | .063 | .063 |
| 1.75 | .083 | .059 | .066 | .063 | .063 |
| 8 | .096 | .073 | .066 | -- | -- |
| 12 | .096 | .070 | .066 | -- | -- |

### EXAMPLE 5

### Preparation of Formulation E

EDTA and buffer were prepared as set forth in Example 1.

The formulation was prepared as set forth in Example 1, except that only one concentration (100 mg/ml) was used. The non-ionic surfactant used was Pluronic 127 (BASF). The results of this experiment are set forth in Table 6. This experiment demonstrates that Pluronic 127 at concentrations from 0.01 weight percent to 1.0 weight percent provides stability against agitation for IL-1ra formulations.

**TABLE 6**

| **0.001 to 1.0% (by weight) Pluronic F127 IL-1ra 100 mg/ml** | | | | | |
|---|---|---|---|---|---|
| **Time (Hours)** | **Weight percent of Pluronic F127** | | | | |
| | **0.001** | **0.01** | **0.1** | **0.5** | **1.0** |
| 0 | .065 | .057 | .056 | .060 | .063 |
| 1.75 | .059 | .049 | .049 | .066 | .064 |
| 8 | .215 | .071 | .059 | -- | -- |
| 12 | .170 | .072 | .059 | -- | -- |

### EXAMPLE 6

### Preparation of Formulation F

EDTA and buffer were prepared as set forth in Example 1.

The formulation was prepared as set forth in Example 1, except that only one
concentration (100 mg/ml) was used. The non-ionic surfactant used was PEG 8000 (Spectrum).
The results of this experiment are set forth in Table 7. This experiment demonstrates that PEG 8000 at concentrations about 1.0 weight percent provides stability against agitation for IL-lra formulations.

**TABLE 7**

| **1.0% and 2.0% (by weight) PEG 8000 IL-1ra 100 mg/ml** | | |
|---|---|---|
| **Time (Hours)** | **Weight percent of PEG 8000** | |
| | **1.0** | **2.0** |
| 0 | .068 | 0.135 |
| 4 | 0.117 | 0.483 |
| 6 | 0.131 | 0.569 |
| 10 | 0.140 | 0.709 |

### EXAMPLE 7

### Preparation of Formulation G

EDTA and buffer were prepared as set forth in Example 1.

The formulation was prepared as set forth in Example 1, except that only one
concentration (100 mg/ml) was used. The non-ionic surfactant used was PEG 300 (Spectrum).
The results of this experiment are set forth in Table 8. This experiment demonstrates that PEG 300 at concentrations about 1.0 weight percent provides stability against agitation for IL-lra formulations.

**TABLE 8**

| **1.0% and 2.0% (by weight) PEG 300 IL-1ra 100 mg/ml** | | |
|---|---|---|
| **Time (Hours)** | **Weight percent of PEG 300** | |
| | **1.0** | **2.0** |
| 0 | .068 | 0.064 |
| 4 | 0.107 | 0.107 |
| 6 | 0.127 | 0.120 |
| 10 | 0.140 | 0.133 |

## Claims

1. A pharmaceutical composition comprising:
(a) interteukin-1 receptor antagonist in amounts from 20 to 200 mg/ml and
(b) the non-ionic surfactant Tween 80, said non-ionic surfactant having a concentration of 0.01-1.0% by weight.

2. A pharmaceutical composition comprising:
(a) interleukin-1 receptor antagonist in an amount of 100 mg/ml and
(b) a non-ionic surfactant selected from the group Tween 20, Pluronic 108, Pluronic F68 and Pluronic 127, said non-ionic surfactant having a concentration of 0.01-1.0% by weight, or a non-ionic surfactant selected from the group of PEG 8000 or PEG 300, said non-ionic surfactant having a concentration of about 1% by weight.

3. The pharmaceutical composition of any of claims 1 or 2, wherein the interleukin-1 receptor antagonist and the non-ionic surfactant is in a weight ratio of about 100 to 1.

4. The pharmaceutical composition of any of claims 1 or 2, wherein the interleukin-1 receptor antagonist and the non-ionic surfactant is in a weight ratio of about 1000 to 1.

5. The pharmaceutical composition of any of claims 1 or 2, wherein the interleukin-1 receptor antagonist and the non-ionic surfactant is in a weight ratio of about 10,000 to 1.

6. The pharmaceutical composition of claim 2, wherein said non-ionic surfactant is Tween 20.

7. The pharmaceutical composition of claim 1 wherein said Tween 80 is at a concentration of 0.1%.

8. The pharmaceutical composition of claim 2 wherein the non-ionic surfactant is a pluronic, selected from the group Pluronic 108, Pluronic F68 and Pluronic 127.

9. The pharmaceutical composition of any of claims 1 or 2, further comprising a buffer selected from the group comprising a phosphate buffer, a citrate buffer and an acetate buffer.

## Patentansprüche

1. Pharmazeutische Zusammensetzung, umfassend:
(a) einen Interleukin-1-Rezeptor-Antagonisten in Mengen von 20 bis 200 mg/ml und
(b) das nicht-ionische Tensid Tween 80, wobei das genannte nicht-ionische Tensid eine Konzentration von 0,01 - 1,0 Gew.-% besitzt.

2. Pharmazeutische Zusammensetzung umfassend:
(a) einen Interleukin-1-Rezeptor-Antagonisten in einer Menge von 100 mg/ml und
(b) ein nicht-ionisches Tensid ausgewählt aus der Gruppe Tween 20, Pluronic 108, Pluronic F68 und Pluronic 127, wobei das genannte nicht-ionische Tensid eine Konzentration von 0,01 - 1,0 Gew.-% besitzt, oder ein nicht-ionisches Tensid ausgewählt aus der Gruppe von PEG 8000 oder PEG 300, wobei das genannte nicht-ionische Tensid eine Konzentration von etwa 1 Gew.-% besitzt.

3. Pharmazeutische Zusammensetzung gemäß einem der Ansprüche 1 oder 2, wobei der Interleukin-1-Rezeptor-Antagonist und das nicht-ionische Tensid in einem Gewichtsverhältnis von etwa 100 zu 1 vorliegen.

4. Pharmazeutische Zusammensetzung gemäß einem der Ansprüche 1 oder 2, wobei der Interleukin-1-Rezeptor-Antagonist und das nicht-ionische Tensid in einem Gewichtsverhältnis von etwa 1000 zu 1 vorliegen.

5. Pharmazeutische Zusammensetzung gemäß einem der Ansprüche 1 oder 2, wobei der Interleukin-1-Rezeptor-Antagonist und das nicht-ionische Tensid in einem Gewichtsverhältnis von etwa 10000 zu 1 vorliegen.

6. Pharmazeutische Zusammensetzung gemäß Anspruch 2, wobei das genannte nicht-ionische Tensid Tween 20 ist.

7. Pharmazeutische Zusammensetzung gemäß Anspruch 1, wobei das genannte Tween 80 in einer Konzentration von 0,1 % vorliegt.

8. Pharmazeutische Zusammensetzung gemäß Anspruch 2, wobei das nicht-ionische Tensid ein Pluronic ist, ausgewählt aus der Gruppe Pluronic 108, Pluronic F68 und Pluronic 127.

9. Pharmazeutische Zusammensetzung gemäß einem der Ansprüche 1 oder 2, weiterhin umfassend einen Puffer, ausgewählt aus der Gruppe bestehend aus einem Phosphatpuffer, einem Citratpuffer und einem Acetatpuffer.

## Revendications

1. Composition pharmaceutique comprenant :
(a) un antagoniste du récepteur de l'interleukine-1 en quantités de 20 à 200 mg/ml et
(b) le surfactant non ionique Tween 80, ledit surfactant non ionique ayant une concentration de 0,01 à 1,0% en poids .

2. Composition pharmaceutique comprenant :
(a) un antagoniste du récepteur de l'interleukine-1 en une quantité de 100 mg/ml et
(b) un surfactant non ionique choisi dans le groupe du Tween 20, du Pluronic 108, du Pluronic F68 et du Pluronic 127, ledit surfactant non ionique ayant une concentration de 0,01 à 1,0% en poids, ou un surfactant non ionique choisi dans le groupe du PEG 8000 ou du PEG 300, ledit surfactant non ionique ayant une concentration d'environ 1% en poids.

3. Composition pharmaceutique selon l'une quelconque des revendications 1 ou 2, dans laquelle l'antagoniste du récepteur de l'interleukine-1 et le surfactant non ionique sont dans un rapport pondéral d'environ 100 sur 1.

4. Composition pharmaceutique selon l'une quelconque des revendications 1 ou 2, dans laquelle l'antagoniste du récepteur de l'interleukine-1 et le surfactant non ionique sont dans un rapport pondéral d'environ 1000 sur 1.

5. Composition pharmaceutique selon l'une quelconque des revendications 1 ou 2, dans laquelle l'antagoniste du récepteur de l'interleukine-1 et le surfactant non ionique sont dans un rapport pondéral d'environ 10000 sur 1.

6. Composition pharmaceutique selon la revendication 2, dans laquelle ledit surfactant non ionique est le Tween 20.

7. Composition pharmaceutique selon la revendication 1, dans laquelle le Tween 80 est à une concentration de 0,1%.

8. Composition pharmaceutique selon la revendication 2, dans laquelle le surfactant non ionique est un Pluronic, choisi dans le groupe du Pluronic 108, du Pluronic F68 et du Pluronic 127.

9. Composition pharmaceutique selon l'une quelconque des revendications 1 ou 2, comprenant en outre un tampon choisi dans le groupe comprenant un tampon phosphate, un tampon citrate et un tampon acétate.
